(19) Europäisches Patentamt
European Patent Office
Office européen des brevets

(11) Publication number: **0 207 580**
**B1**

(12) # EUROPEAN PATENT SPECIFICATION

(45) Date of publication of patent specification: **26.07.89**

(51) Int. Cl.[4]: **C 07 C 103/46,** C 07 C 102/00

(21) Application number: **86301131.8**

(22) Date of filing: **19.02.86**

(54) **Process for the synthesis of N-acetyl amino acids from olefins, acetamide and syngas.**

(30) Priority: **05.04.85 US 720248**
**05.04.85 US 720226**
**05.04.85 US 720227**
**05.04.85 US 720228**

(43) Date of publication of application:
**07.01.87 Bulletin 87/02**

(45) Publication of the grant of the patent:
**26.07.89 Bulletin 89/30**

(84) Designated Contracting States:
**BE CH DE FR GB IT LI NL**

(56) References cited:
**DE-A-2 826 676**
**DE-A-3 242 374**

(73) Proprietor: **TEXACO DEVELOPMENT CORPORATION**
**2000 Westchester Avenue**
**White Plains New York 10650 (US)**

(72) Inventor: **Lin, Jiang Jen**
**2617 Oak Meadow Drive**
**Round Rock Texas 78664 (US)**

(74) Representative: **Wood, Anthony Charles et al**
**Urquhart-Dykes & Lord 91 Wimpole Street**
**London W1M 8AH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 207 580 B1

**Description**

This invention relates to the synthesis of N-acylamino acids from olefinically unsaturated hydrocarbons, amides and syngas.

More particularly this invention uses a bimetallic rhodium-cobalt catalyst to synthesize N-acylamino acids from various types of olefinically unsaturated hydrocarbon, amides and synthesis gas, with very high yield using moderate pressures and temperatures.

Early attempts were unsuccessfully made to synthesize alpha-amino acids, or derivatives thereof, by reacting a Schiff's base or a nitrile with carbon monoxide and hydrogen. [*Bull. Chem. Soc. Japan* 33 (160) 78]

US—A—3 766 266 discloses a cobalt-catalysed method of producing an N-acyl-alpha-amino acid from an aldehyde, a carboxylic acid amide and carbon monoxide at a temperature of 10° to 300°C and a pressure of at least 500 atm. (50 MPa).

In *Chem. Comm.* 1540 (1971), Wakamatsu, et al disclose a cobalt-catalyst reaction which gives various N-acyl amino-acids from an aldehyde, an amide and carbon monoxide. When benzaldehyde was used as the starting aldehyde, no corresponding alpha-phenyl-substituted amino acid was obtained. Instead, an imine was obtained by a simple "amination" reaction.

An article by Parnaud, et al., in *Journal of Molecular Catalysts,* 6 (1979) 341—350, discusses the reaction wherein N-acyl-alpha-amino acids are produced by reacting an aldehyde, CO and an amide in the presence of dicobalt octacarbonyl.

In amidocarbonylation, the aldehyde can be added as such, or generated in situ from allyl alcohol, alkyl halides, oxiranes, alcohols or olefins, followed by the reaction with an amide and carbon monoxide to produce an N-acyl-alpha-amino acid.

US—A—3 996 288 discloses that when an alcohol or certain of its ester derivatives is held at 50°C to 200°C and 10 to 50 atm. (1 to 50 MPa) in the presence of hydrogen, carbon monoxide, the amide of a carboxylic acid and a carbonylation catalyst, an aldehyde having one or more carbon atom than the alcohol or ester is formed in good yield. If the amide has at least one active hydrogen atom on its amide nitrogen, it further reacts with the aldehyde and carbon monoxide to form an N-acylamino acid.

Hirai, et al. in *Tetrahedron Letters,* Vol. 23, No. 24, pp. 2491—2494, (1982) discuss a process for combining the transition metal-catalyzed isomerization of allyl alcohol to aldehyde with cobalt-catalyzed amidocarbonylation, to provide a route to N-acyl-alpha-amino acids.

A recent review article by Ojima in *Journal of Organometallic Chemistry,* 279 (1985), 203—214, discussed the synthesis of N-acetyl-alpha-amino acids from (a) the isomerization-amidocarbonylation of allylic alcohols, (b) the isomerization-amidocarbonylation of oxiranes, and (c) the hydroformylation-amidocarbonylation of trifluoropropene. The hydroformylation-amidocarbonylation of trifluoropropene demonstrated a surprising regioselectivity for products *1* and *2*.

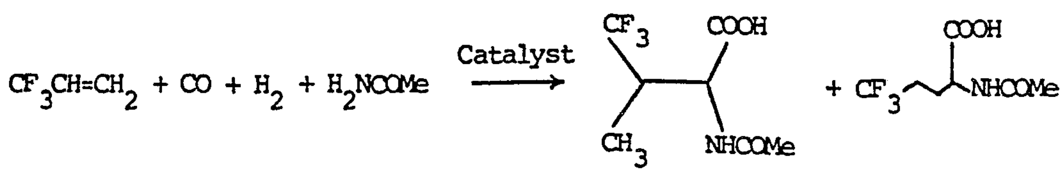


*1* *2*

N-acetyltrifluorovaline (*1*) (94%) and N-acetyltrifluoronorvaline (*2*) (96%) were obtained in high yields by using $CO_2(CO)_8$—$Rh_6(CO)_{16}$ and $Co_2(CO)_8$ as catalysts respectively. This shows a surprising difference in product when using $Co_2(CO)_8$ as opposed to $Co_2(CO)_8$—$Rh_6(CO)_{16}$ catalysts, in the special case of the fluorolefin substrate.

DE—A—2826676 provides a process for the production of N-acylamino acids in which an olefinically-unsaturated hydrocarbon, an amide, carbon monoxide and hydrogen are reacted in a solvent at a temperature of at least 50°C and a pressure of at least 3.5 MPa in the presence of a catalyst comprising rhodium and cobalt.

The present invention is characterised in that the catalyst comprises a cobalt carbonyl and a rhodium compound comprising a triphenyl phosphine ligand.

The results of the present invention, using especially $Co_2(CO)_8$—HRh $(CO)(PPh_3)_3$ for various olefinically-unsaturated hydrocarbons, are significantly different from those of the two last-mentioned documents in the following respects:

(1) The presence of $HRh(CO)(PPh_3)_3$ stabilizes dicobalt octacarbonyl, and allows the reaction to proceed predictably at a low temperature in comparison with dicobalt octacarbonyl alone.

(2) The combination of $HRh(Co)(PPh_3)_3$ and $CO_2(CO)_8$ constitutes a bimetallic complex, as shown by the presence of new infrared absorption in the carbonyl region and by the catalyst behaviour attributable to the complex.

(3) The combined $HRh(CO)(PPh_3)_3$—$Co_2(CO)_8$ catalyst performs the reaction under milder reaction conditions.

(4) Various rhodium species in the Rh—Co complexes affect the reactivity and regioselectivity.

(5) In some cases, dicobalt octacarbonyl performs the same catalyst activity at higher temperatures but simultaneously produced the reduced hydrocarbon as by-product. This disadvantage was overcome by using a suitable Rh—Co catalyst.

In one embodiment of the invention, solid or liquid N-acylamino acids are obtained from alpha olefins or internal olefins. Yields of the N-acylamino acids are as high as 80 to 90%, and a linearity of 95% is observed using very mild reaction. The products from internal olefins could be valuable as surfactants, chelating agents or gas treating agents. The N-acylamino acids obtained from the alpha olefins can be useful as surfactants.

In another embodiment the olefinically unsaturated hydrocarbon is a diene, and the products comprise novel amino acids with an olefin functionality, and regio-isomer mixtures of novel bis-(N-acetylamino acids). These compounds may be intermediate for surfactants and chelating agents.

In another embodiment, the olefinically unsaturated hydrocarbon can be styrene or a substituted styrene.

In a further embodiment, the olefinically unsaturated hydrocarbon can be a vinylidene compound.

The products may be solid or liquid at room temperature, depending on the type of olefin used as a reactant.

The N-acylamino acids from alpha olefins are generally solids at room temperature. In contrast the N-acylamino acids from $C_{14}$ and $C_{18}$ internal olefins are generally liquid at room temperature.

These latter derivatives are predominantly branched alkyl-N-acylamino acids, useful for surfactants, chelating agents and gas treating agents.

In general, the alpha-olefin or internal olefin can have the formula:

$$R.CH=CH_2 \text{ or } R.CH=CH.R$$

in which R is alkyl, or the two groups R can together represent the carbon atoms necessary to complete an alicyclic ring.

The reaction for producing solid linear alkyl-N-acetylamino acids from alpha olefins can be represented by equation (1):

(1) 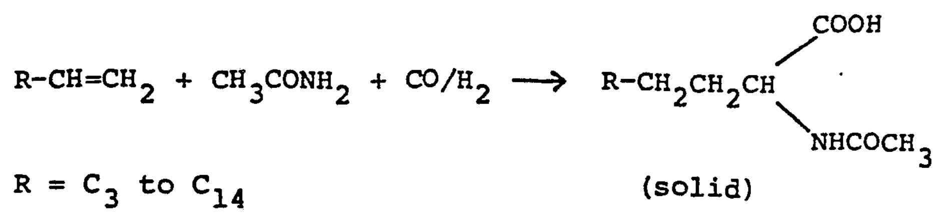


The reaction for producing liquid branched alkyl-N-acetylamino acids from internal olefins can be represented by equation (2):

(2) 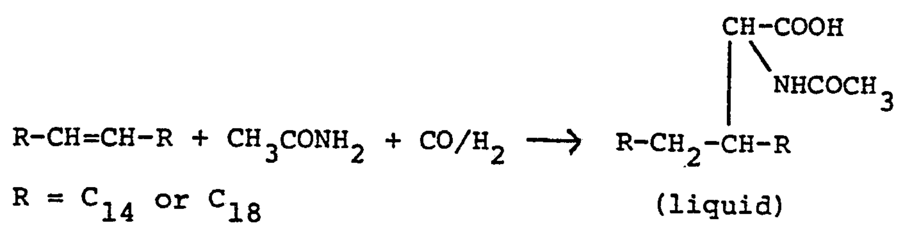


The reactions for producing solid amino acid derivatives from acyclic and cyclic internal olefins can respectively be represented by equations (3) and (4):

(3) 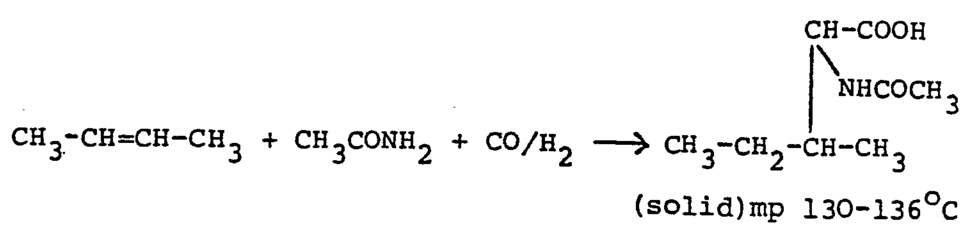

(4) 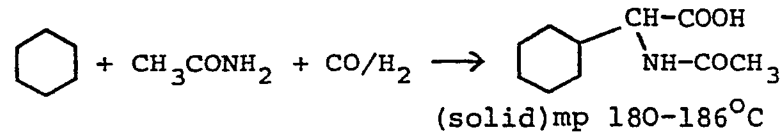

(solid)mp 180-186°C

When the olefinically unsaturated hydrocarbon is a diene, it may be unsymmetrical, or it may be symmetrical.

Amino acids bearing an olefin functionality are obtained from unsymmetrical dienes having two double bonds, each with different reactivity. Suitable dienes include 4-vinyl-1-cyclohexene, vinylnorbornene, bicyclopentadiene, 1,6-octadiene and limonene.

Preferred dienes are unsymmetrical dienes such as 4-vinylcyclohexene, limonene bicyclopentadiene or 1,6-octadiene.

The reaction can be demonstrated by the following equations:

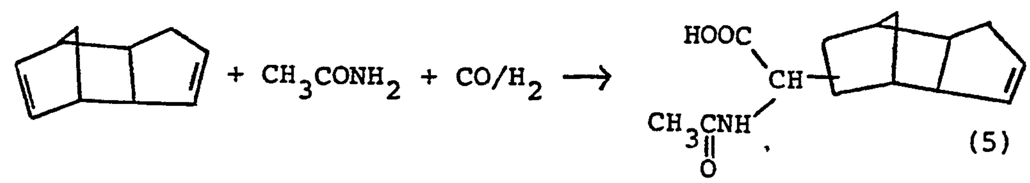

(5)

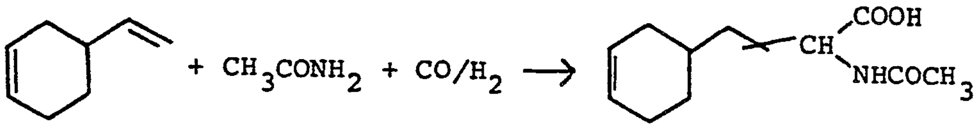

(6)

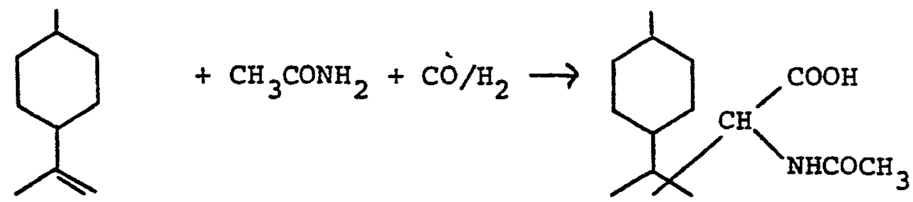

(7)

Regio-isomer mixtures of bis-(N-acetylamino acids) are synthesized from symmetrical dienes, in which the two double bonds have identical reactivities.

Suitable dienes include 1,3-butadiene, 1,7-octadiene, norbornadiene, 1,3-cyclohexadiene, and 1,5-cyclooctadiene.

Preferred dienes include 1,7-octadiene or 1,3-butadiene. Synthesis of the novel bis amino acids proceeds, for example, according to the following reactions (8) and (9):

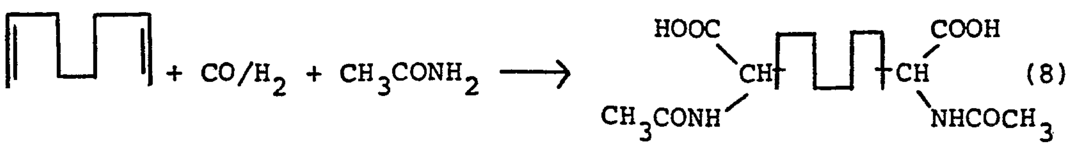

(8)

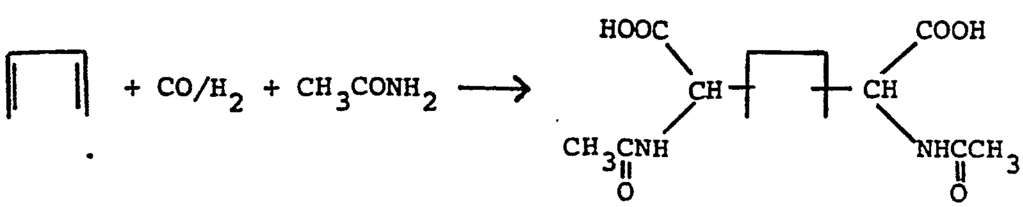

(9)

In another embodiment, the olefinically unsaturated hydrocarbon is styrene or a substituted styrene having the formula

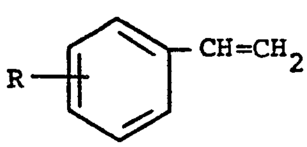

in which R is alkyl.

The reaction can best be represented by the following equation (10):

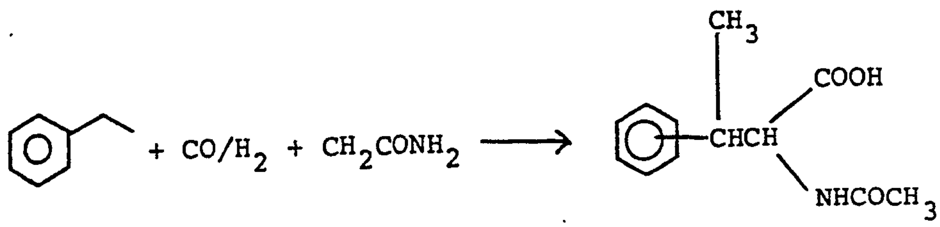

(10)

In a further embodiment, the olefinically unsaturated hydrocarbon is a vinylidene compound having the formula

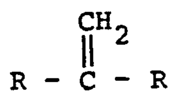

in which each R is alkyl.

This reaction can best be represented by the following equation (11):

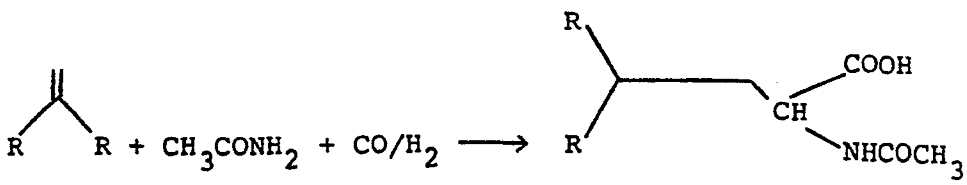

(11)

Recovery of the N-acetylamino acids from the reaction product can be carried out in any convenient or conventional manner such as by distillation, extraction, filtration or crystallization. In the reactions illustrated above, the products were recovered by a simple extraction procedure, and identificed by HMR.

The catalyst system suitable for the practice of this invention comprises the specific bimetallic rhodium-cobalt catalysts optionally in a substantially inert solvent.

In the catalyst system, the rhodium-compound and cobalt compound are believed to be in complex equilibrium during amidocarbonylation. The controlled experiments represented by the Examples show that the presence of both Rh and Co is essential for consistent production of the desired results. This catalyst system provides the following important advantages over the use of cobalt alone:

(1) It gives higher yields and selectivities of the N-acetylamino acid products under milder conditions than can be obtained with a catalyst comprising only a cobalt compound dispersed in a solvent.

(2) It is possible to employ relatively mold operating conditions, especially in the embodiment using internal olefins to produce solid N-acetylamino acids.

(3) It was possible to obtain from alpha olefins a 95% linear product. This degree of linearity is specifically observed with the alpha olefin 1-tetradecene as a feedstock and is affected by certain species of rhodium compound.

(4) Mixtures of $HRh(CO)(PPh_3)_3$ and $Co_2(CO)_8$ form stable catalytically active complexes which are easily recovered from the reaction mixture.

In the process of the invention the rhodium compound contains a triphenylphosphine ($PPh_3$) ligand. Compounds which work well in this respect include those where the rhodium is added to the reaction zone as a carbonyl, hydrocarbonyl or substituted carbonyl species wherein the substituted group is triphenylphosphine. The preferred compound is hydridorhodium tris(triphenylphosphine)carbonyl, $HRh(CO)(PPh_3)_3$.

It is worthwhile to note that some other rhodium species have adverse effects on catalyst activity as shown, in the comparative examples. It is assumed that these rhodium species might form an unactive species, or might deactivate cobalt carbonyl, although the mechanism for this effect is unclear.

The cobalt compound may take many different forms. For instance, the cobalt may be added to the reaction mixture in the form of a variety of cobalt carbonyls.

The cobalt carbonyls may be tetracobalt dodecacarbonyl or dicobalt octacarbonyl. The preferred cobalt-containing compound is dicobalt octacarbonyl.

The alpha olefins can have the following structure:

$$R{-}CH{=}CH_2$$

and the internal olefins can have the structure:

R—CH=CH—R

The R-group can be any alkyl, such as methyl, ethyl, hexyl or octyl, either the normal or branched isomers. The preferred alpha olefins include propylene, 1-octene, 1-decene, 1-dodecene and 1-tetradecene. Particularly good results are obtained using 1-tetradecene.

The olefin can also be an internal olefin such as a $C_{14}$ to $C_{18}$ internal olefin including, but not limited to 7-tetradecene, 9-octadecene or 2-butene and cycloalkenes, such as cyclohexene or cyclopentene.

The starting olefinically unsaturated hydrocarbon can be styrene or a substituted styrene having the structure:

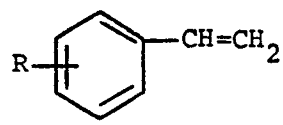

R can be any alkyl, such as methyl, ethyl, hexyl or octyl, at any of the ortho-, meta- or para-positions. The preferred compound is styrene.

The olefinically unsaturated hydrocarbons can alternatively be vinylidene compounds having the formula:

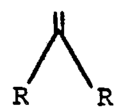

where the groups R, which can be the same or different, are alkyl, such as methyl, ethyl, hexyl or octyl. The preferred vinylidene compounds include $C_{14}$ vinylidene and isobutylene. Particularly good results are obtained using isobutylene.

Suitable amides that are useful in the amidocarbonylation reaction have the general structure:

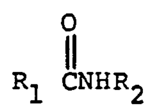

where $R_1$ and $R_2$, which may be the same or different, are each substituted or unsubstituted aryl, alkyl, arylalkyl, alkylaryl, or hydrogen, e.g. methyl, ethyl, butyl, n-octyl, phenyl, benzyl or chlorophenyl. Examples of suitable amides include acetamide, benzamide, formamide, N-methylformamide, lauramide and N-methylbenzamide. The preferred amide is acetamide.

The carbon monoxide employed need not satisfy particular purity requirements, although catalyst contaminants should be avoided if the reaction is intended to continue over an extended period. Particularly in continuous operations, but also in batch experiments, the carbon monoxide and hydrogen gas may also be used in conjunction with up to 10% by volume of one or more other gases. These other gases may include one or more inert gases that are not inert but which may, or may not, undergo reaction under carbon monoxide hydrogenation conditions, such as carbon dioxide, hydrocarbons, such as methane, ethane and propane, ethers, such as dimethyl ether, methyl ethyl ether and diethyl ether and alkanols, such as methanol.

As characterized above, this process is operated as a homogeneous liquid phase mixture. The reaction is preferably operated in an inert solvent. Preferred inert solvents are those which permit at least partial solution of the cobalt and rhodium catalyst precursors, the amide and the olefinically unsaturated hydrocarbon. These are generally polar solvents, of the ester, ether, ketone, amide, sulfoxide or aromatic hydrocarbon type, for example.

Methyl and ethyl acetate are examples of suitable solvents. Other polar solvents are ethers, such as p-dioxan, methyl tertiary-butyl ether, methyl tertiary-amyl ether or tetrahydrofuran, tertiary amides, such as dimethyl formamide, dimethyl sulfoxide and ethylene carbonate.

The preferred solvent is ethyl acetate.

The amino acid products are often insoluble in the solvent phase. This permits separation of the rhodium catalyst which may dissolve into the solvent phase, with or without prior acidification.

In all these syntheses, in order to achieve a high degree of selectivity, the amounts of carbon monoxide, olefin and amide present in the reaction mixture should be sufficient at least to satisfy the stoichiometry of the desired reaction forming N-acetylamino acid as shown in Equations 1 to 10 above. An excess of carbon monoxide over the stoichiometric amount is desirable.

The quantity of rhodium compound and cobalt compound to be used in the catalyst may vary. The

process is conducted in the presence of catalytically effective quantities of the active rhodium and cobalt compounds which give the desired products in reasonable yield. The reaction proceeds when employing as little as 0.01 weight % of the rhodium compound or even less, along with as little as 0.1 weight% of the cobalt compound, based on the total weight of the reaction mixture. The upper concentration is dictated by a variety of factors, including catalyst cost, partial pressures of carbon monoxide and hydrogen, operating temperature, etc. Amounts of rhodium compound from 0.01 to 1.0 weight % in conjunction with from 0.1 to 10 weight % cobalt compound, based on the total weight of the reaction mixture, are generally desirable in the practice of this invention.

Particularly superior results are obtained when the rhodium to cobalt mol ratio is from 1.0:1.0 to 1.0:1000.

The operating conditions may vary over a wide range. The reaction temperature is generally from 50 to 300°C. The preferred temperature is from 80 to 150°C. The pressure may range from 3.5 to 20 MPa, but may be higher, depending on the olefinically unsaturated hydrocarbon. In the embodiment using internal olefins, it appears that higher selectivities are obtained when operating at moderate pressures, in the range from 7 to 25 MPa. In the embodiment using alpha olefins, very good yields are observed using very mild pressures and temperatures, in the range of 5.5 to 14 MPa and 100 to 120°C respectively. With dienes, preferred conditions are 7 to 25 MPa and 70 to 150°C. With styrenes, preferred conditions are 7 to 25 MPa and 80 to 150°C. With vinylidene compounds, preferred conditions are 5.5 to 25 MPa and 80 to 150°C.

The amidocarbonylation reaction is best conducted in a carbon monoxide-rich atmosphere, although some hydrogen gas should also be present in order to achieve maximum cobalt catalyst activity. The hydrogen to carbon monoxide mol ratio in the reactor may be varied, for example, within the range from 20:1 to 1:20, but preferably it should be relatively rich in carbon monoxide and the $H_2$:CO ratio should be in the range 5:1 to 1:5.

The main products of the synthesis using alpha olefins are solid N-acylamino acids, for example N-acetyl-alpha-amino-isovaleric acid, alpha-(n-dodecyl)-N-acetyl-glycine, alpha-N-acetylamino-tetradecanoic acid, alpha-(n-tetradecyl)-N-acetyl-glycine, alpha-(decyl)-N-acetyl-glycine, and alpha-(n-octyl)-N-acetyl-glycine. Also formed are significant amounts of aldehyde. Each of these products, including by products, can be recovered from the reaction mixture by conventional means, e.g. crystallization or filtration.

The main products from internal olefins are, for example, alpha-(sec-butyl)-N-acetylglycine, alpha-(n-tetradecyl-7)-N-acetylglycine and alpha-(cyclohexyl)-N-acetylglycine.

The major products from unsymmetrical dienes are amino acids with an olefin functionality. The main product from dicyclopentadiene and acetamide has the structure:

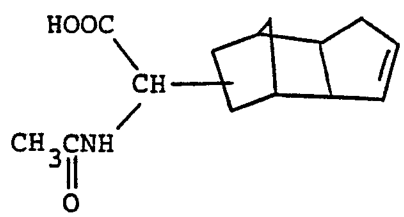

and may be formed in significant quantities of as much as 78% yield.

The main products from symmetrical dienes, i.e. bis-(N-acetylamino) acids, are formed in significant quantites. They have the structure:

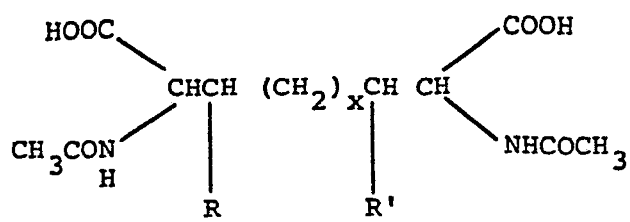

Where x=1 to 10

R, R'=methyl or H

The main products from styrenes are phenyl substituted amino acids. The main product from styrene itself, beta-phenyl-N-acetyl-alpha-amino acid, will be formed in significant quantities.

The main products from vinylidene compounds are solid N-acetylamino acids, represented by, for example alpha-isobutyl-N-acetylglycine and alpha-(2-hexyl-octyl)-N-acetylglycine.

The process of the invention can be conducted in a batch, semi-continuous or continuous manner. The catalyst can be initially introduced into the reaction zone batchwise, or it may be continuously or intermittently introduced into such a zone during the course of the synthesis reaction. Operating conditions can be adjusted to optimize the formation of the desired amino acid product, and said material may be recovered by methods known to the art, such as filtration, recrystallization distillation, extraction and the like. A fraction rich in the catalyst components may then be recycled to the reaction zone, if desired, and additional products generated.

The products have been identified in this work by one or more of the following analytical procedures: gas-liquid phase chromatography (glc), gas chromatography/infared spectroscopy (GC/IR), nuclear magnetic resonance (nmr) and elemental analysis, or a combination of these techniques. Analyses have for the most part, been by molar weight; all temperatures are in degrees centigrade and all pressures in Megapascals (MPa).

The yield (mol %) of N-acylamino acid derivative in this synthesis using an olefin is estimated using the formula:

$$\frac{\text{Mols of N-acylamino acids obtained}}{\text{Mols of olefinically unsaturated compound charged}} \times 100\%$$

Example 1

A glass-lined reactor equipped with rocking device was charged with dicobalt octacarbonyl (0.34 g), hydrido-tris-(triphenylphosphino) carbonyl rhodium (0.046 g), acetamide (5.9 g, 0.1 mol), and ethyl acetate (10.0 g). The reactor was flushed with a 1:1 $CO/H_2$ mixture. Propylene (10 g) was added to the reactor, and the pressure was brought up to 13.9 MPa with 1:1 $CO/H_2$ and 18.0 MPa with pure CO. The system was heated to ca. 120°C. During the heating process the maximum pressure reached 21.8 MPa. After two hours reaction time, the reactor was cooled to room temperature. The solid product (11.0 g) and liquid (22.0 g) were recovered. H-nmr analysis showed that the solid was a mixture of the N-acetyl-amino acids N-acetyl-alpha-aminovaleric acid *3* and N-acetyl-alpha-amino-isovaleric acid *4* at 2:1 molar ratio. The combined yield of these two isomers was calculated to be 70%, based on acetamide charged. The liquid solution contained compounds of Structures *5* and *6*:

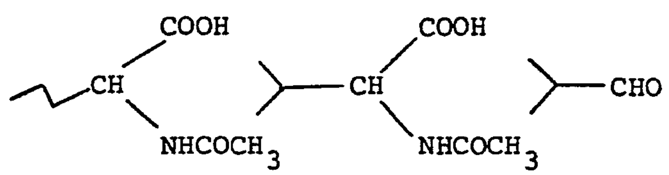 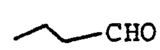

3 4 5 6

Aldehydes *5* and *6* were present at 1.6 to 1.0 mol ratio. The isomer distribution, i.e. linear vs. branched products, for *3:4* was the reverse of that for *6:5*. Two possible explanations were proposed: (1) the reaction of linear aldehyde *6* with acetamide and CO is faster than the reaction of aldehyde *5*; or (2) the aldehydes are not the intermediates for amino acid formation. The actual mechanism for N-acetylamino acid formation from olefins is unknown.

Example 2 (Comparative)

Example 1 was repeated, omitting the dicobalt octacarbonyl, the reactor being pressurised to 18.0 MPa which $CO/H_2$ (molar ratio 1.6:1). The maximum pressure reached 128.4 MPa and a significant consumption of gas was noted. After 2 hours, the reactor was cooled to room temperature. The product solution (25.4 g) was analyzed. H-nmr showed that only aldehyde compounds were produced.

It was thereby demonstrated that the amidocarbonylation of aldehydes into N-acetylamino acids requires the presence of cobalt catalyst.

Example 3 (Comparative)

Example 2 was repeated, using dicobalt octacarbonyl (0.34 g, 1 mmol) in place of the rhodium compound. The maximum pressure reached 22.85 MPa. After two hours, the reactor was allowed to cool to room temperature. The solid material (6.0 g) and the liquid material (9.0 g) were recovered. H-nmr analyses indicated the solid was unreacted acetamide, and no N-acetylamino acid was obtained.

Dicobalt octacarbonyl is ineffective for propylene hydroformylation, although the recovered liquid solution showed 1.0% concentration of cobalt (ca. 80% based on cobalt charged.

Example 4

A glass lined reactor equipped with a rocking device was charged with hydrido-tris(triphenylphosphino)carbonyl rhodium (0.046 g), dicobalt octacarbonyl (0.34 g), 1-dodecene (8.4 g, 0.05 mmol), acetamide (3.0 g, 0.05 mol) and p-dioxane (10.0 g). The reactor was sealed and flushed with $CO/H_2$ mixture (1:1 molar ratio). The system was pressurized with $CO/H_2$ mixture (1:1) to 0.8 MPa and heated to 100°C. The pressure was raised to 15.25 MPa (with $CO/H_2$=1:1). The conditions were held for four hours. During the process, a pressure drop of 2.75 MPa was noted. After cooling to room temperature, the reactor was opened and solid and liquid product mixtures were recovered. The mixtures were filtered and 12.8 g of solid material was obtained. The solid was analyzed by H-nmr to be alpha-(n-dodecyl)-N-acetyl-glycine (or alpha-N-acetylamino-tetradecanoic acid).

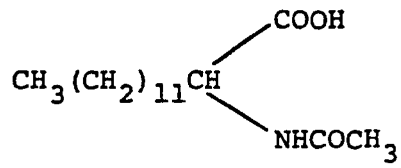

The yield was ca. 74%. There were no other isomeric N-acetylamino acids in the products or starting materials. The linearity of the product was estimated to be >95% based on H-nmr analysis.

Example 5 (Comparative)

The same experimental procedures and reaction conditions as in Example 4 were used, except that no $HRh(CO)(PPh_3)_3$ was added. The recovered mixtures contained only 1-dodecene, acetamide and no N-acetyl-alpha-amino acid was observed.

The experiment was repeated, and the same results were reported. It was concluded that, at the conditions used, the presence of $HRh(CO)(PPh_3)_3$ is essential for hydroformylation/amidocarbonylation of alpha olefins.

Example 6

The experimental procedures of Examples 1 to 5 were followed, employing: $HRh(CO)(PPh_3)_3$, (0.046 g), $Co_2(CO)_8$ (0.34 g), acetamide (3.0 g), 1-tetradecene (98 g) and ethyl acetate (10.0 g) at 13.9 MPa, $CO/H_2=1:1$ and 100°C for 4 hours. The recovered materials (25.4 g) contained some crystalline solid. After filtration, a white solid powder was obtained (13.8 g). H-nmr analysis indicated the solid product had the structure *8*; alpha (n-tetradecyl)-N-acetyl-glycine. Its branched isomer was *not* detected by H-nmr.

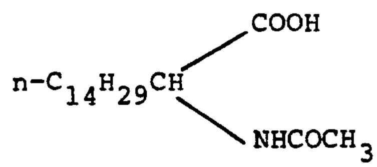

*8*

The yield of compound *8* was ca. 89%. The product contained 45.8 ppm of rhodium and 400 ppm of cobalt.

Example 7

The typical procedures of the previous Examples were followed, employing $HRh(CO)(PPh_3)_3$ (0.023 g), $Co_2(CO)_8$ (0.17 g), acetamide (3.0 g), 1-tetradecene (9.8 g) and ethyl acetate (15.0 g), at 13.9 MPa, $CO/H_2=1:1$ and 100°C for 4 hours. The recovered materials contained liquid and crystalline solid. After filtration and washing twice with ethyl acetate, 13.0 g of white powdered solid material was obtained. H-nmr and $C_{13}$ nmr analyses indicated it was the pure form of alpha-(n-tetradecyl-N-acetyl)-glycine, and no isomeric branched amino acids were observed.

The yield of this desired product was ca. 83%. The solid product contained only <5 ppm Rh and 170 ppm Co.

Example 8 (Comparative)

Typical experimental procedures were employed, except the rhodium species was in the form of rhodium (0.5%) on an alumina support.

The reactor was charged with Rh (0.5%) on Al (0.10 g), $Co_2(CO)_8$ (0.17 g), 1-tetradecene (9.8 g), acetamide (3.0 g) and ethyl acetate (15.0 g). The reaction conditions were 13.9 MPa, $CO/H_2=1:1$, 100°C and 2 hours. The recovered product solution was analyzed by H-nmr, showing that unreacted 1-tetradecene and acetamide were the major components, and no aldehyde was in the amino acid product.

The example demonstrates the importance of the rhodium species.

Example 9 (Comparative)

Typical experimental procedures were used, except that $Rh(acac)_3$ was the rhodium source, and dicobalt octacarbonyl was the cobalt source.

$Co_2(CO)_8$ (0.17 g), $Rh(acac)_3$ (0.010 g, 0.025 mmol), 1-tetradecene (9.8 g), acetamide (3.0 g) and ethyl acetate (15.0 g) were reacted at 100°C and 13.9 MPa, $CO/H_2=1:1$, for 4 hours. The recovered solution (23.0 g) and solid (5.0 g) were analyzed by H-nmr. There was *no* alkyl N-acetylglycine, the desired product. Instead, the bisamidal compound was the major component of the solid material.

This showed that the combination of $Rh(acac)_3/Co_2(CO)_8$ is not suitable for amino acid synthesis.

Example 10 (Comparative)

Typical experimental procedures were used, except that dicobalt octacarbonyl was the catalyst, p-dioxane was the solvent, and *no* rhodium was involved.

A glass-lined autoclave was charged with dicobalt octacarbonyl (0.125 g, 0.37 mmol), 1-tetradecene (9.8 g, 0.05 mol), acetamide (3.0 g, 0.05 mol), and p-dioxane (15.0 g). The reactor was flushed with syngas and pressurized to 10.1 MPa with $CO/H_2$=1:1 mixture. The system was heated to 110°C and held at 12.2 MPa for two hours, then cooled at room temperature, and the excess gas was vented off. The two-layered liquid materials were recovered, 18.0 g and 6.8 g. The top layer contained 1-tetradecene and p-dioxane and the bottom layer contained acetamide and p-dioxane. Therefore, it was concluded dicobalt octacarbonyl was not a good catalyst for the 1-tetradecene/acetamide reaction under such conditions.

Example 11

A glass lined autoclave was charged with $HRh(CO)(PPh_3)_3$ (0.023 g), $Co_2(CO)_8$ (0.34 g), 1-decene (10.5 g), acetamide (3.0 g) and p-dioxane (15.0 g). The reaction conditions were 100°C, 14.6 MPa, $CO/H_2$=1:1 for four hours.

A homogeneous product solution was obtained (30.3 g). The solid product appeared while setting at room temperature. The solid material (9.0 g) was washed twice by ca. 10 g of cold ethyl acetate, resulting in a light grey solid (8.1 g). H-nmr analysis indicated a structure of alpha-(decyl)-N-acetyl glycine. The yield was ca. 63%.

Analysis of the solid product by atomic absorption showed it contained only 4.5 ppm of rhodium and 4.5 ppm of cobalt.

Example 12

The typical experimental procedures of previous examples were used, employing hydrido-tris(tri-phenylphoshino)carbonyl rhodium (0.046 g), dicobalt octacarbonyl (0.34 g), acetamide (5.9 g, 0.1 mol), 1-octene (8.96 g, 0.08 mole) and ethyl acetate (10.0 g). A pressure of 13.9 MPa, ($CO/H_2$=1:1 mol ratio) was added to the reactor and a temperature of 120°C was maintained. At these conditions the pressure was maintained at 18.0 MPa with CO and kept for two hours. A deep red homogeneous solution (28.3 g) was obtained, which was analyzed by H-nmr. The conversion of 1-octene was 100% and the only product detected was alpha-(n-octyl)-N-acetyl glycine.

Example 13

Typical experimental procedures were used, except a 300 ml stirring autoclave was charged with $HRh(CO)(PPh_3)_3$ (0.06 g), $Co_2(CO)_8$ (0.68 g), 1-tetradecene (9.8 g), acetamide (3.0 g), and ethyl acetate (20.0 g). The reaction conditions were 5.6 MPa, $CO/H_2$=1:1 and 109—112°C for 4 hours. Alkyl-N-acetyl glycine (8.5 g) was obtained. The yield was estimated to be ca. 55%.

This example showed the catalyst was active under the low pressure conditions although the isolated yield was relatively lower.

Examples 14 to 20 demonstrate the use of internal olefins to produce novel liquid amino acid derivatives.

Example 14

A glass-lined reactor was charged with $HRh(CO)(PPh_3)_3$ (0.046 g, 0.05 mmol), $Co_2(CO)_8$ (0.34 g, 1 mmol), acetamide (3.0 g, 51 mmols), 7-tetradecene (9.8 g, 50 mmols), and ethyl acetate (10.0 g). The system was purged of air and pressurized with $CO/H_2$ mixture (1:1 molar ratio) to 0.8 MPa, then heated to 100°C. The pressure was raised to 13.9 MPa and held for four hours at these conditions. The reactor was cooled to room temperature and the excess gas vented. The resulting homogeneous liquid (25.0 g) was recovered. The metal analyses indicated 204 ppm rhodium (ca. 99% of theoretical) and 0.417% cobalt (ca. 91% of theoretical). A portion of the product solution (2.00 g) was subjected to high vacuum to remove ethyl acetate solvent. The viscous liquid product (1.15 g) was analyzed by H-nmr (90 MHz) in various solvents and shown to be the N-acetyl-alkyl-amino acid compound (structure 10). The yield of the product, based on 7-tetradecene, was ca. 85%.

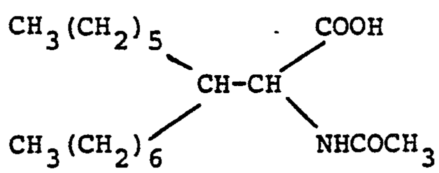

*10*

Example 15

The experimental procedure of Example 14 was repeated, except for using $HRh(CO)(PPh_3)_3$ (0.023 g), $Co_2(CO)_8$ (0.17 g), 9-octadecene (12.7 g), acetaide (3.0 g) and ethyl acetate (8.0 g). The operating conditions were 120°C, 13.9 MPa of $CO/H_2$=1:1, for 4 hours. The resulting homogeneous solution (25.4 g) was analyzed by H-nmr and showed structure *11*.

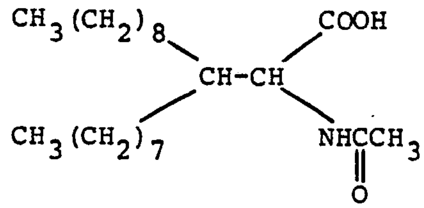

11

Example 16

The experimental procedure of Example 14 was repeated, using $HRh(CO)(PPh_3)_3$ (0.023 g), $Co_2(CO)_8$ (0.34 g), acetamide (2.9 g), 9-octadecene (12.7 g) and ethyl acetate (10.0 g), with 13.9 MPa of $CO/H_2=1:1$ mol ratio at 100°C for 4 hours. The resulting liquid (28.9 g) was analyzed by atomic absorption and contained 3500 ppm of cobalt and 109 ppm of rhodium. A liquid product was recovered after removing the ethyl acetate solvent. The H-nmr showed ca. 80% yield of N-acetyl-alkyl-amino acid. The glc analysis showed that the conversion of 9-octadecene was >95% and the by product was formed with ~14% selectivity.

Example 17 (Comparative)

The experimental procedure of Example 14 was repeated, except for omitting the rhodium compound. The resulting material contained 40.4 ppm of cobalt. The glc analysis showed the recovered 9-octadecene (~90%) in the solution. The presence of $HRh(CO)(PPh_3)_3$ was important at these operating conditions.

Example 18 (Comparative)

Experimental procedures similar to those in Example 17 were employed, using $Co_2(CO)_8$ (0.68 g), 9-octadecene (12.6 g), acetamide (3.0 g) and ethyl acetate (20 g). The operating conditions were 5.6 MPa, 130°C, and $CO/H_2=1:2$ for four hours. The recovered liquid was analyzed by H-nmr, showing ca. 52% yield of N-acetylamino acid and ca. 48% yield of reduced hydrocarbons. It is noted that dicobalt octacarbonyl alone required a higher reaction temperature than 100°C and resulted in a loser yield of the desired product.

Example 19

The reactor was charged with $HRh(CO)(PPh_3)_3$ (0.023 g), $Co_2(CO)_8$ (0.17 g), acetamide (3.0 g), 2-butene (~14.0 g) and ethyl acetate (10.0 g). The conditions were 13.9 MPa, $CO/H_2=1:1$ and 120°C for 4 hours. The resulting product mixtures were filtered to obtain 3.5 g solid and 12.5 g filtrate. The H-nmr showed the solid was the isomeric N-acetyl-amino acid *12*, (m.p. 130—136°C).

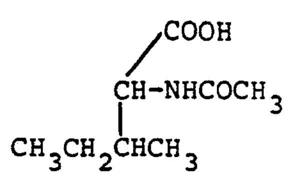

12

The yield was estimated at ca. 34%, based on charged acetamide.

Example 20

Experimental procedures identical to Example 19 were used, except for using $HRh(CO)(PPh_3)_3$ (0.046 g), $Co_2(CO)_8$ (0.34 g), acetamide (3.0 g), cyclohexene (4.1 g, 50 mmol) and ethyl acetate (10.0 g) with reaction conditions of $CO/H_2=1:1$ and 120°C for 4 hours.

The resulting product mixture (19.2 g) was filtered and the solid material (8.5 g) was analyzed by H-nmr, showing structure *13*.

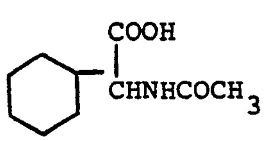

13

The melting point of compound 13 was 180—186°C and the yield was 85%.

Example 21

A glass-lined reactor was charged with hydridocarbonyl-(tris-triphenylphosphine) rhodium (I), $HRh(CO)(PPh_3)_3$ (0.046 g, 0.05 mmol), dicobalt octacarbonyl (0.34 g, 1 mmol), dicyclopentadiene (3.3 g, 25 mmols), acetamide (3.0 g, 51 mmols) and p-dioxane (15.0 g). The reactor was sealed and purged of air with a mixture of $CO/H_2$. Then the pressure was raised to 13.9 MPa with $CO/H_2$ mixture (1:1 molar ratio) and heated to 100°C. A maximum pressure of 15.6 MPa was recorded. After two hours the system was cooled to

room temperature and excess gas was vented. The resulting product solution (brown liquid, 23.1 g) was analyzed by atomic absorption, and contained 4260 ppm of cobalt (~85% of theoretical) and 186 ppm of rhodium (83% of theoretical). H-nmr analysis identified structure *14* as the major product in ca. 78% yield based on charged dicyclopentadiene.

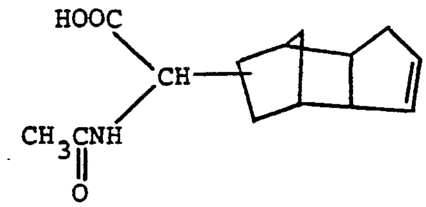

*14*

Example 22

The experimental procedures of Example 21 were employed, using $HRh(CO)(PPh_3)_3$ (0.046 g), $Co_2(CO)_8$ (0.34 g), acetamide (3.0 g), 4-vinyl-1-cyclohexene (5.4 g, 0.50 mmols) and ethyl acetate (10.0 g) at 13.9 MPa, $CO/H_2$=1:1 molar ratio and 100°C for 4 hours. The product solution (22.3 g) was recovered. H-nmr analysis showed the presence of compound *15*.

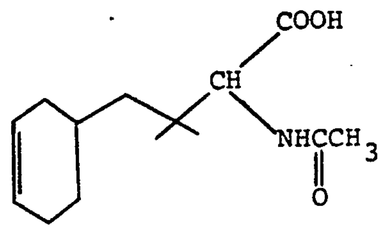

*15*

Example 23

The experimental procedures of Example 22 were employed, except that the pressure was 7.0 MPa. The product solution (20.6 g) contained only compound *16* and no compound *15*.

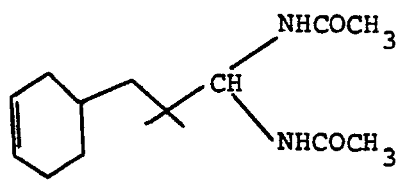

*16*

Presumably, compound *16* is the intermediate to compound *1* and required additional carbonylation.

Example 24

The experimental procedures of Example 21 were employed, using $HRh(CO)(PPh_3)_3$ (0.046 g), $Co_2(CO)_8$ (0.34 g), 4-vinyl-1-cyclohexene (65 g), acetamide (3.0 g) and ethyl acetate (10.0 g), at 13.9 MPa, $CO/H_2$=1:1 and 100°C for 4 hours. The resulting product mixture (22.2 g) was analyzed by H-nmr. The presence of compound *15* and the aldehyde by product was detected.

Example 25

The experimental procedures of Example 21 were employed, using $HRh(CO)(PPh_3)_3$ (0.046 g), $Co_2(CO)_8$ (0.34 g), acetamide (2.9 g), limonene (6.8 g) and ethyl acetate (10 g) at 13.9 MPa, $CO/H_2$=1:1 and 100°C for 4 hours. The resulting product solution (21.3 g) was analyzed by H-nmr. The conversion of limonene was 100%. Two products were detected at ca. 1:1 molar ratio.

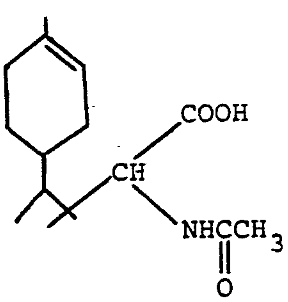

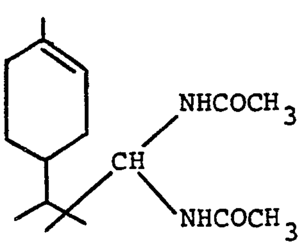

*17* *18*

Example 26 (Comparative)

Procedures similar to those used in Examples 21 to 25 were employed, using $Co_2(CO)_8$ (0.34 g), acetamide (3.0 g), dicyclopentadiene (3.3 g) and p-dioxane at 13.9 MPa of $CO/H_2$=1:1, and 100°C for 2 hours. The resulting light brown solution (21.9 g) contained mostly recovered dicyclopentadiene (>80% recovery).

Example 27

A glass-lined autoclave was charged with hydridocarbonyl(tris-triphenylphosphino)rhodium (I), $HRh(CO)(PPh_3)_3$ (0.046 g, 0.05 mmol), $Co_2(CO)_8$ (0.34 g, 1 mmol), acetamide (3.0 g, 51 mmol), 1,7-octadiene (2.8 g, 25 mmols) and ethyl acetate (15.0 g). The autoclave was purged of air with a mixture of $CO/H_2$ at 1:1 ratio and pressurized to 0.8 MPa with $CO/H_2$. Then the reactor was heated to 100°C and the pressure was increased to 13.9 MPa. After 2 hours the system was cooled to room temperature, excess gas was vented and the reaction mixture (23.4 g) was filtered to obtain 7.5 g of solid product. H-nmr analysis of the solid product showed the presence of isomer mixtures:

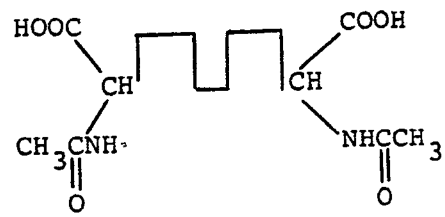

*19*

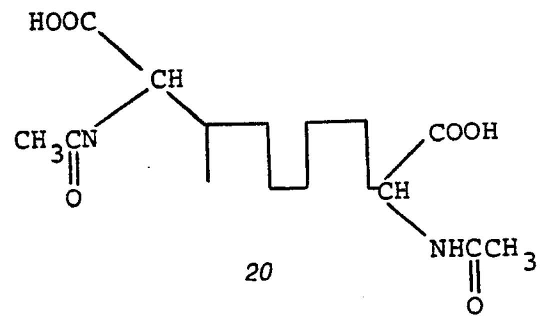

*20*

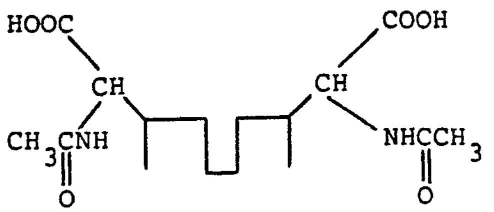

*21*

The product distribution for linear to branched (i.e. *19:21*) was estimated to be 2:1, based on the H-nmr integration. The isolated yield of bis-amino acid was 30%.

Example 28

The experimental procedures of Example 21 were employed, using $HRh(CO)(PPh_3)_3$ (0.046 g, 0.05 mmol), $Co_2(CO)_8$ (0.34 g, 1 mmol), acetamide (3.0 g, 51 mmols), 1,7-octadiene (5.6 g, 50 mmols) and p-dioxane (15.0 g) at 13.9 MPa of $CO/H_2$ (1:1) and 100°C for 2 hours. The homogeneous product solution (28.1 g) was analyzed by H-nmr. The product distribution, based on 1,7-octadiene charged, was 44% for N-acetylamino acid and 34% for aldehyde intermediates.

Example 29

The experimental procedures of Example 28 were employed, using $HRh(CO)(PPh_3)_3$ (0.046 g, 0.05 mmol), $Co_2(CO)_8$ (0.34 g, 1 mmol), acetamide (3.0 g, 51 mmols), p-dioxane (15.0 g) and 1,3-butadiene (6 g, 110 mmols) at 13.9 MPa of $CO/H_2$ (1:1 molar ratio) and 100°C for 4 hours. A homogeneous product solution was recovered (22.1 g). The H-nmr showed the mixture of bis-amino acid derivatives in 45% yield, based on acetamide reactant.

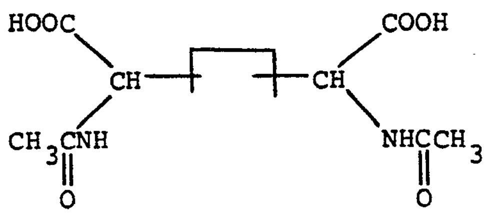

Example 30

The reactor was charged with $HRh(CO)(PPh_3)_3$ (0.046 g), $Co_2(CO)_8$ (0.34 g), acetamide (3.0 g), 1,7-octadiene (2.8 g) and ethyl acetate (15.0 g).

The system was pressurized with $CO/H_2$ at a 1:2 mixture to 8.4 MPa then with CO to 13.9 MPa (totally). It was heated at 100°C for 2 hours. The resulting product (24.9 g) was analyzed by H-nmr, showing the presence of bis-amidal compound and no N-acetylamino acid.

Example 31

A glass-lined reactor was charged with $HRh(CO)(PPh_3)_3$ (0.092 g, 0.10 mmol), dicobalt octacarbonyl (0.34 g, 1.0 mmol), styrene (5.2 g, 0.05 mol), acetamide (3.0 g, 0.05 mmol) and p-dioxane (10 g). The reactor was purged of air with $CO/H_2$ mixture, then pressurized to 3.5 MPa with $CO/H_2$ (1:2 molar ratio). The system was heated to 100°C and the pressure was raised to 13.9 MPa with $CO/H_2$ mixture (1:2). After 4 hours the reactor was cooled to room temperature. A deep dark homogeneous solution (19.8 g) was obtained (1.3 g wt gain based on material charged). A solid material (2.35 g) appeared in the bottom of the product solution after standing overnight. The solid product was analyzed by H-nmr to be: bis(acetylamino)propyl benzene (A + B) at approximately 21% yield. The liquid product contained two aldehyde products.

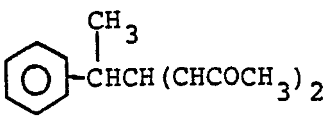

(A)

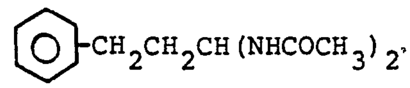

(B)

Example 32

The experimental procedures of Example 31 were used, except that the solvent was ethyl acetate and the molar ratio of $CO/H_2$ was 1:1.

The reactor was charged with $HRh(CO)(PPh_3)_3$ (0.46 g, 0.050 mmol), dicobalt octacarbonyl (0.34 g, 1.0 mmol), styrene (5.2 g, 0.05 mol), acetamide (3.0 g, 0.05 mol) and ethyl acetate (15.0 g). The reactor was purged of air and pressurized to 0.8 MPa with $CO/H_2$ mixture (1:1 molar ratio). The system was heated to 100°C and the pressure was raised to 13.9 MPa. After 4 hours the reactor was cooled to room temperature. The excess gas was vented. A dark black solution (25.5 g) was recovered with approximately 2.0 g weight gain, based on starting material charged. After standing at room temperature, a precipitate appeared. The mixture was filtered. 8.2 g of light brown solid was obtained. The H-nmr showed the solid product was beta-phenyl-N-acetyl-alpha-amino acid (C). The yield was ca. 75%, based on styrene charged. The product was further identified by its silyl derivative (D) using BSTFA, (N,O-bis-(trimethylsilyl)-trifluoroacetamide) reagent.

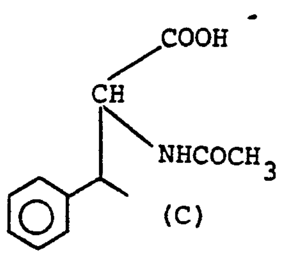

(C)

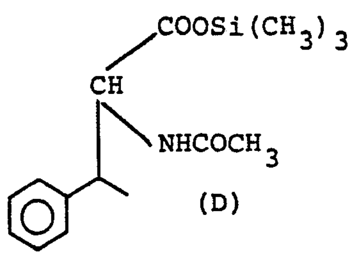

(D)

Example 33 (Comparative)

The procedures of Examples 31 and 32 were used, except no rhodium catalyst was employed.

The reactor was charged with dicobalt octacarbonyl (0.34 g, 1 mmol), styrene (5.2 g, 0.05 mol), acetamide (3.0 g, 0.05 mol) and ethyl acetate (15.0 g). The reaction conditions were 13.9 MPa of $CO/H_2$=1:1 molar ratio and 100°C for 4 hours. The reaction product contained only ethyl benzene and aldehyde product. No N-acetyl amino acid was obtained.

This Example shows that the rhodium catalyst enhances the reaction to achieve the amino acid product. Otherwise, the reaction product (ethyl benzene) will be formed.

Example 34

A 300 ml stirred reactor was charged with $HRh(CO)(PPh_3)_3$ (0.046 g, 0.05 mmol), dicobalt octacarbonyl (0.68 g, 2.0 mmol), styrene (5.2 g, 0.05 mol), acetamide (3.0 g, ca. 0.05 mmol) and ethyl acetate (20 g). The reactor was purged of air and pressurized to 0.8 MPa. After heating to 100°C, the system was pressurized with $CO/H_2$ (1:1 mixture) to 5.6 MPa. During 4 hours reaction time, 0.97 MPa of syngas pressure uptake was recorded. The final product mixture contained 6.0 g solid and 23.3 g liquid. The solid was analyzed by H-nmr, shown to be N-acetyl-beta-phenyl-amino acid.

The yield was 55%, based on styrene charged.

This Example showed the Rh/Co bimetallic catalyst 'was' active even at 5.6 MPa.

Example 35 (Comparative)

Example 34 was repeated, except that no rhodium catalyst was present.

The reactor was charged with $Co_2(CO)_8$ (0.68 g), styrene (5.2 g), acetamide (3.0 g) and ethyl acetate (20 g). The conditions were 100°C, 5.6 MPa and 4 hours. The recovered liquid product contained no N-acetyl-beta-phenyl-amino acid.

Examples 36 and 37 demonstrate production of a solid form of N-acetylamino acid from isobutylene and $C_{14}$ vinylidene respectively. Example 38 demonstrates the poor results observed when a vinylidene compound and a low pressure are used. Example 39 demonstrates the poor results observed when a cobalt-containing compound was used alone, thus emphasizing the importance of bimetallic system.

Example 36

A glass-lined reactor was charged with $HRh(CO)(PPh_3)_3$ (0.046 g), $Co_2(CO)_8$ (0.34 g), acetamide (3.0 g) and ethyl acetate (10.0 g). The reactor was sealed and purged with synthesis gas. Isobutylene (ca. 15 g) was pressurized into the reactor. 1.1 MPa of syngas ($CO/H_2$=1:1 molar ratio) was pressurized and the reactor was heated to 100°C, then the pressure was brought up to 13.9 MPa with $CO/H_2$=1:1 mixture. The reaction was held for four hours and 13.9 MPa was maintained by the addition of $CO/H_2$ through a gas cylinder. After cooling and venting the excess gas, 27.9 g of product was obtained. The solid product (7.4 g) was obtained through filtration and drying. Analysis showed the material was pure alpha-isobutyl-N-acetylglycine, melting point 128—135°C.

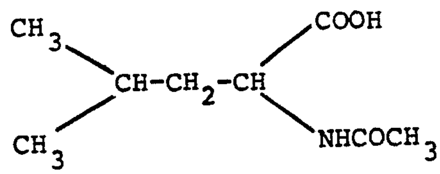

The yield was estimated to be 83%, based on acetamide charged.

Example 37

Following the experimental procedures of Example 36, the mixture of $HRh(CO)(PPh_3)_3$ (0.023 g), $Co_2(CO)_8$ (0.17 g, 0.5 mmol), $C_{14}$ vinylidene (9.8 g, 0.05 mol), acetamide (3.0 g, 0.05 mol) and ethyl acetate (15 g) was reacted at 13.9 MPa, $CO/H_2$=1:1 and 100°C for 4 hours. The resulting products were filtered and the solid material (10.1 g), mp 93—98°C, was analyzed by H-nmr and was found to be

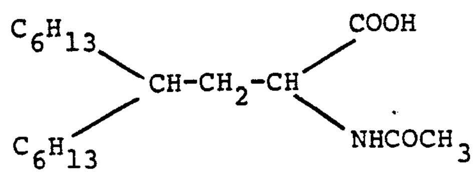

The yield was 66%, based on olefin charged.

Example 38

A mixture of $HRh(CO)(PPh_3)_3$ (0.023 g), $Co_2(CO)_8$ (0.68 g), $C_{14}$ vinylidene (9.8 g), acetamide (3.0 g) and ethyl acetate (20 g) was charged into a 300 ml reactor. The operating conditions were 5.6 MPa of $CO/H_2$=1:1 and 100°C for four hours. The resulting materials (two layers of liquid ca. 1:2 ratio of upper to bottom) were analysed and no N-acetylamino acid was observed.

Example 39 (Comparative)

The experimental procedures of Example 38 were duplicated, except that no $HRh(CO)(PPh_3)_3$ was used.

The mixture of $Co_2(CO)_8$ (0.17 g), $C_{14}$ vinylidene (9.8 g), acetamide (3.0 g) and ethyl acetate was reacted at 13.9 MPa of $CO/H_2$=1:1 and 100°C for 4 hours. The resulting product solution showed >90% of $C_{14}$ vinylidene recovered.

## Claims

1. A process for the production of N-acylamino acids by reacting an olefinically-unsaturated hydrocarbon, an amide, carbon monoxide and hydrogen in a solvent at a temperature of at least 50°C and a pressure of at least 3.5 MPa in the presence of a catalyst comprising rhodium and cobalt, characterised in

that the catalyst comprises a cobalt carbonyl and a rhodium compund containing a triphenyl phosphine ligand.

2. A process according to claim 1 characterised in that the catalyst comprises hydridotris-(triphenylphosphine) carbonyl rhodium HRh(CO) $(PPh_3)_3$, and dicobalt octacarbonyl $Co_2$ $(CO)_8$.

3. A process according to claim 1 or 2 characterised in that the olefinically unsaturated hydrocarbon is an alpha-olefin or internal olefin having the formula:

$$R.CH{=}CH_2 \text{ or } R.CH{=}CH.R$$

in which R is alkyl, or the two groups R can together represent the carbon atoms necessary to complete an alicyclic ring.

4. A process according to claim 3 characterised in that the olefinically unsaturated hydrocarbon is propylene, 1-octene, 1-decene, 1-dodecene or 1-tetradecene.

5. A process according to claim 3 characterised in that the olefinically unsaturated hydrocarbon is 7-tetradecene or 9-octadecene.

6. A process according to claim 3 characterised in that the olefinically unsaturated hydrocarbon is cyclohexene or cyclopentene.

7. A process according to claim 1 or 2 characterised in that the olefinically unsaturated hydrocarbon is styrene or a substituted styrene having the formula:

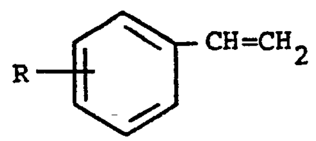

in which R is alkyl.

8. A process according to claim 1 or 2 characterised in that the olefinically unsaturated hydrocarbon is an unsymmetrical diene having two olefin functions of different reactivity.

9. A process according to claim 8 characterised in that the diene is 4-vinylcyclohexene, limonene, bicyclopentadiene, 1,6-octadiene or vinyl norbornene.

10. A process according to claim 1 or 2 characterised in that the olefinically unsaturated hydrocarbon is a symmetrical conjugated or non-conjugated diene.

11. A process according to claim 10 characterised in that the diene is 1,3-butadiene, 1,7-octadiene, norbornadiene, 1,3-cyclohexadiene or 1,5-cyclohexadiene.

12. A process according to claim 1 or 2 characterised in that the olefinically unsaturated hydrocarbon is a vinylidene compound having the formula

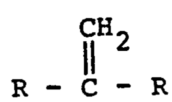

in which each R is alkyl.

13. A process according to claim 12 characterised in that the vinylidene compound is isobutylene or $C_{14}$ vinylidene.

**Patentansprüche**

1. Verfahren zur Herstellung von N-Acylaminosäuren durch Umsetzung eines olefinisch ungesättigten Kohlenwasserstoffes, eines Amins, Kohlenmonoxid und Wasserstoff in einem Lösungsmittel bei einer Temperatur von mindestens 50°C und einem Druck von mindestens 3,5 MPa in Gegenwart eines aus Rhodium und Kobalt bestehenden Katalysators, dadurch gekennzeichnet, daß der Katalysator aus einem Kobaltcarbonyl und einer einen Triphenylphosphinliganden enthaltenden Rhodiumverbindung besteht.

2. Verfahren nach Anspruch 1, dadurch gekennzeichnet, daß der Katalysator aus Hydridotris-(triphenylphosphin)-carbonyl-rhodium $HRh(CO)(PPh_3)_3$ und Dikobalt-octacarbonyl $Co_2(CO)_8$ besteht.

3. Verfahren nach Anspruch 1 oder 2, dadurch gekennzeichnt, daß der olefinisch ungesättigte Kohlenwasserstoff ein alpha-Olefin oder mittelständiges Olefin der Formel:

$$R.CH{=}CH_2 \text{ oder } R.CH{=}CH.R$$

worin R für Alkyl steht oder die beiden Gruppen R zusammen die zur Ergänzung zu einem alizyklischen Ring erforderlichen Kohlenstoffatome darstellen, ist.

4. Verfahren nach Anspruch 3, dadurch gekennzeichnet, daß der olefinisch ungesättigte Kohlenwasserstoff Propylen, 1-Octen, 1-Decen, 1-Dodecen oder 1-Tetradecen ist.

5. Verfahren nach Anspruch 3, dadurch gekennzeichnet, daß der olefinisch ungesättigte Kohlenwasserstoff 7-Tetradecen oder 9-Octadecen ist.

6. Verfahren nach Anspruch 3, dadurch gekennzeichnet, daß der olefinisch ungesättigte Kohlenwasserstoff Cyclohexen oder Cyclopenten ist.

7. Verfahren nach Anspruch 1 oder 2, dadurch gekennzeichnet, daß der olefinisch ungesättigte Kohlenwasserstoff Styrol oder ein substituiertes Styrol der Formel

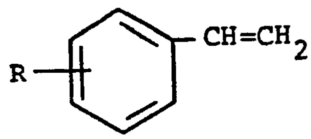

worin R für Alkyl steht, ist.

8. Verfahren nach Anspruch 1 oder 2, dadurch gekennzeichnet, daß der olefinish ungesättigte Kohlenwasserstoff ein unsymmetrisches Dien mit zwei Olefinfunktionen verschiedener Reaktionsfähigkeit ist.

9. Verfahren nach Anspruch 8, dadurch gekennzeichnet, daß das Dien 4-Vinylcyclohexen, Limonen, Bicyclopentadien, 1,6-Octadien oder Vinylnorbornen ist.

10. Verfahren nach Anspruch 1 oder 2, dadurch gekennzeichnet, daß der olefinisch ungesättigte Kohlenwasserstoff ein symmetrisches konjugiertes oder nicht konjugiertes Dien ist.

11. Verfahren nach Anspruch 10, dadurch gekennzeichnet, daß das Dien 1,3-Butadien, 1,7-Octadien, Norbornadien, 1,3-Cyclohexadien oder 1,5-Cyclohexadien ist.

12. Verfahren nach Anspruch 1 oder 2, dadurch gekennzeichnet, daß der olefinisch ungesättigte Kohlenwasserstoff eine Vinylidenverbindung der Formel

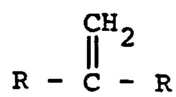

worin R jeweils für Alkyl steht, ist.

13. Verfahren nach Anspruch 12, dadurch gekennzeichnet, daß die Vinylidenverbindung Isobutylen oder $C_{14}$-Vinyliden ist.

**Revendications**

1. Procédé de préparation de N-acylamino acides en faisant réagir un hydrocarbure à insaturation oléfinique, un amide, de l'oxyde de carbone et de l'hydrogène, dans un solvant, à une température d'au moins 50°C et sous une pression d'au moins 3,5 MPa en présence d'un catalyseur comprenant du rhodium et du cobalt, caractérisé en ce que le catalyseur comprend un cobalt carbonyle et un composé du rhodium contenant de la triphénylphosphine comme ligand.

2. Procédé suivant la revendication 1, caractérisé en ce que le catalyseur comprend du rhodium hydridotris-(triphénylphosphine)carbonyle $HRh(CO)(PPh_3)_3$, et du dicobalt octacarbonyle $Co_2(CO)_8$.

3. Procédé suivant la revendication 1 ou 2, caractérisé en ce que l'hydrocarbure à insaturation oléfinique est une alpha-oléfine ou une oléfine interne répondant à la formule:

$$R.CH{=}CH_2 \text{ ou } R.CH{=}CH.R$$

dans laquelle R est un groupe alkyle, ou bien les deux groupes R peuvent représenter ensemble les atomes de carbone nécessaire pour compléter un cycle alicyclique.

4. Procédé suivant la revendication 3, caractérisé en ce que l'hydrocarbure à insaturation oléfinique est le propylène, le 1-octène, le 1-décène, le 1-dodécène ou le 1-tétradécène.

5. Procédé suivant la revendication 3, caractérisé en ce que l'hydrocarbure à insaturation oléfinique est le 7-tétradécène ou le 9-octadécène.

6. Procédé suivant la revendication 3, caractérisé en ce que l'hydrocarbure à insaturation oléfinique est le cyclohéxène ou le cyclopentène.

7. Procédé suivant la revendication 1 ou 2, caractérisé en ce que l'hydrocarbure à insaturation oléfinique est le styrène ou un styrène substitué répondant à la formule:

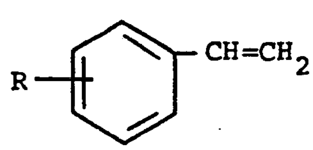

dans laquelle R est un groupe alkyle.

8. Procédé suivant la revendication 1 ou 2, caractérisé en ce que l'hydrocarbure à insaturation oléfinique est un diène asymétrique ayant deux fonctions oléfines de réactivité différente.

9. Procédé suivant la revendication 8, caractérisé en ce que le diène est le 4-vinylcyclohéxène, le limonène, le bicyclopentadiène, le 1,6-octadiène ou le vinyl norbornène.

10. Procédé suivant la revendication 1 ou 2, caractérisé en ce que l'hydrocarbure à insaturation oléfinique est un diène symétrique conjugué ou non conjugué.

11. Procédé suivant la revendication 10, caractérisé en ce que le diène est le 1,3-butadiène, le 1,7-octadiène, le norbornadiène, le 1,3-cyclohexadiène ou le 1,5-cyclohexadiène.

12. Procédé suivant la revendication 1 ou 2, caractérisé en ce que l'hydrocarbure à insaturation oléfinique est un composé vinylidénique répondant à la formule:

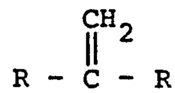

dans laquelle chacun des R est un groupe alkyle.

13. Procédé suivant la revendication 12, caractérisé en ce que le composé vinylidénique est l'isobutylène ou un vinylidène en $C_{14}$.